# EUROPEAN PATENT SPECIFICATION

(11) **EP 0 339 751 B1**
(45) Date of publication and mention of the grant of the patent: **29.05.1996**
(21) Application number: 89201074.5
(22) Date of filing: 25.04.1989
(51) Int. Cl.: C12P 37/04, C12R 1/02, C12R 1/64

(54) **Process for the enzymatic preparation of beta-lactams**
Verfahren zur enzymatischen Herstellung von beta-Lactamen
Procédé de préparation enzymatique de bêta-lactames

(30) Priority: 26.04.1988 EP 88200811
(43) Date of publication of application: 02.11.1989
(73) Proprietor: GIST-BROCADES N.V., NL-2611 XT Delft (NL)
(72) Inventor: van der Laken, Cornelis Jacobus, NL-2318 NG Leiden (NL)
(74) Representative: Visser-Luirink, Gesina, Dr.

(56) References cited:
- FR-A- 2 119 736
- FR-A- 2 132 271
- FR-A- 2 213 932
- FR-A- 2 310 757
- CHEMICAL ABSTRACTS, vol. 69, no. 25, 16 December 1968, Columbus, OH (US); p. 10046, no. 107066c
- CHEMICAL ABSTRACTS, vol. 93, no. 15, 13 October 1980, Columbus, OH (US); p. 547, no. 148118k

## Description

This invention relates to a process for the enzymatic preparation of a β-lactam derivative with a 6-D-α-aminophenylacetamido side chain by subjecting a phenylglycine derivative and the corresponding 6-amino β-lactam derivative to the action of one or more enzymes of microbial origin.

Generally, this type of semi-synthetic penicillin is prepared by chemical methods, for example by reacting 6-aminopenicillanic acid (6-APA), having its carboxyl group usually protected, with a side chain precursor, followed by removal of the protecting group by hydrolysis. For example, ampicillin (6-D-α-aminophenylacetamidopenicillanic acid) can be prepared by reacting 6-APA, having a suitably protected carboxyl group, with D-phenylglycinechloride.hydrochloride, followed by hydrolysis.

German Offenlegungschrift No. 2163792 describes the enzymatic preparation of aminopenicillin compounds by reacting a D-phenylglycine derivative with (unprotected) 6-APA in the presence of enzymes from a microorganism belonging to the genera Acetobacter, Xanthomonas, Mycoplana, Protaminobacter or Aeromonas. In Austrian Patent No. 243986, the enzyme from a Pseudomonas species has been mentioned as a catalyst for the reaction of a D-phenylglycine derivative and 6-APA. In Dutch patent application No. 7009138, the enzyme of the microorganism Flavobacterium EF-44-102 has been described to catalyze the same reaction.

German Offenlegungschrift No. 2621618 describes the enzymatic preparation of aminopenicillin compounds, by reacting a D-phenylglycine derivative with 6-APA in the presence of a mycelial preparation from microorganisms of the genera Aphanocladium or Cephalosporium.

In all these patent documents the temperature applied during the examples disclosed is 25°C or higher, especially 37°C and 28°C.

It has now surprisingly been found that this type of enzymatic reaction can advantageously be carried out by using a temperature of 0-15°C, preferably of 5-15°C. A considerable higher yield of about 88% at a reaction temperature of 5°C instead of about 73% at a reaction temperature of 30°C can be obtained. No attempts have been made to optimize the reaction times by the choice of other enzymes, for instance. Also the enzymatic synthesis of ampicillin by reacting 6-APA and a D,L-phenylglycine derivative preferably is carried out at a temperature of 0-15°C, more preferably of 5-15°C, with essentially the same result. By using the racemate of the phenylglycine derivative instead of the pure D-form, a costly conversion of the D,L-phenylglycine derivatives into the D-phenylglycine reagent before reacting with 6-APA is no longer necessary.

Suitable phenylglycine (PG) derivatives for the purpose of this invention are, for example, phenylglycine and amides and esters of PG (PG-X), such as the (lower alkyl) esters and N(lower alkyl) amides. Lower alkyl is defined throughout this application as C₁₋₆ straight and branched alkyl chains. Preferred PG derivatives are the methyl and ethyl ester and the N(methyl) and N(ethyl) amide of phenylglycine.

Suitable β-lactam compounds are, for example, penicillanic acids or salts or esters thereof.

Suitable salts of the penicillanic acids include conventional non-toxic salts such as an alkali metal salt (e.g. sodium salt, potassium salt, etc.) and an alkaline earth metal salt (e.g. calcium salt, magnesium salt, etc.), an ammonium salt, an organic base salt (e.g. trimethylamine salt, triethylamine salt, pyridine salt, picoline salt, dicyclohexylamine salt, N,N'-dibenzylethylenediamine salt, etc.), an organic acid salt (e.g. acetate, maleate, tartrate, methanesulfonate, cinnamate, p-chlorocinnamate, benzenesulfonate, formate, toluenesulfonate, etc.), an inorganic acid salt (e.g. hydrochloride, hydrobromide, hydroiodide, sulfate, phosphate, etc.) or a salt with an amino acid (e.g. arginine, aspartic acid, glutamic acid, etc.) and the like.

Suitable esters of the penicillanic acids include conventional, non-toxic esters, such as the methyl ester, ethyl ester, propyl ester, butyl ester, benzyl ester, optionally substituted, such as the diphenylmethyl ester and the 4-nitrobenzyl ester, etc. etc.

Suitable enzymes are, for example, the enzymes of microbial origin that catalyze the reactions of 6-amino β-lactams and a D-PG derivative. Preferably, the enzymes of the microorganisms Acetobacter pasteurianum and Xanthomonas citrii are used for this reaction using a D,L-PG derivative.

The reaction conditions depend on various parameters, in particular the reagents, reaction time, temperature and enzyme concentration. The preferred conditions can be readily determined by the man skilled in the art. Suitable reaction times are from several minutes to several hours, in particular from about 0.5 hour to about 2 hours. Suitable enzyme concentrations are from about 0.5 U/ml to about 10 U/ml.

It surprisingly appeared that the enzymatic reactions are highly stereospecific. The reaction with a D,L-PG derivative yields the D-form of the β-lactam with a 6-α-aminophenylacetamido side chain in more than 96% relative to the total yield. The suitable enzymes are believed to bring about the following reaction 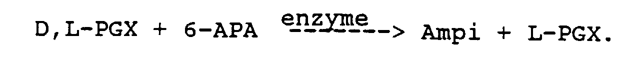 Further separation of the product enantiomers and of L-PGX, if desired, can be achieved by methods known per se, for example by crystallisation.

The invention is further illustrated by the following non-limitating examples.

### Example I

### Enzyme extract from Acetobacter pasteurianum ATCC 6033

The strain Acetobacter pasteurianum ATCC 6033 was grown according to the method described by Kato (Kato, K. et al., Agric. Biol. Chem. 44, (1980) 1067-1074). After 40 hours at 28°C the culture broth (5 l) was centrifuged for 15 minutes at 11,000 g. The pellet (200 g wet weight) was suspended in 1 1 0.1 M TRIS (tris-(hydroxymethyl)amino methane buffer)/HCl, pH 8.0. Next 1 g lysozyme and 4 g EDTA were added and the solution was stirred for 24 hours at 20°C. 1% octanol v/v and 1% Triton® x-100 v/v were added. After 2 hours at 20°C the pH was reduced to 7.0 and 4 g MgCl₂ and 1 mg DNase were added. After stirring for 24 hours at 20°C the lysed broth was centrifuged for 10 minutes at 11,600 g.

The supernatant was diluted four times with water, the pH was reduced to 6.0 and 15 g dry SP-Sephadex cation exchange was added. After stirring for 1 hour, the Sephadex material was filtered and washed with 500 ml of 10 mM 4-morpholino ethanesulphonic acid (MES) buffer, pH 6.0. The enzyme activity was eluted by a solution of a high salt concentration (0.5 M NaCl in MES buffer, pH 6.0).

This enzyme concentrate was dialyzed against 10 mM MES with pH 6.0 and used as such throughout all the following Examples. The final enzyme solution had an activity of 18 U/ml. One unit corresponds to the amount of enzyme that hydrolyzes per minute 1 µmole D-phenylglycinemethyl ester into D-PG under standard conditions (30 mM D-PGMe, 100 mM MES, pH 6.3, 30°C).

### Example II

### Temperature dependence of stereospecific synthesis of ampicillin

The enzymatic synthesis was performed with the acylase from Acetobacter pasteurianum (prepared in Example I).

The assay conditions were; pH-stat, pH 6.3, 10 mM 6-APA, 60 mM D,L-PGMe.

Time samples and both isomers were detected by reversed-phase HPLC described by F. Jehl et al., Path. Biol. 31, (1983) 370.
- Detailed description:: column: µ Bondapak C-18, 3.9 x 300 mm
eluent: 10% acetonitrile, 0.2 M ammonium acetate with pH 5.0; 1 ml/min
detection: 254 mm
retention times:
Ampi : 8.1 min
L-Ampi* : 5.4 min
6-APA : 3.7 min
PG : 3.5 min
D,L-PGMe : 9.2 min.

* 6-L-α-aminophenylacetamidopenicillanic acid

In Table I the percentages of Ampi and L-Ampi formed (based on 10 mM 6-APA) at different temperatures are listed.

**TABLE I**

| | | | |
|---|---|---|---|
| TEMP (°C) | ENZYME (U/ml) | AMPI FORMED (%) | L-AMPI FORMED (%) |
| 5 | 13 | 77 | 8 |
| 10 | 6 | 66 | 4 |
| 30 | 1 | 60 | 2 |

### Example III

### Temperature dependence of ampicillin synthesis

For the enzymatic synthesis of ampicillin the acylase from Acetobacter pasteurianum (prepared in Example I) was used.

The assay conditions were: pH-stat, pH 6.3, 10 mM 6-APA, 30 mM D-PGMe.
Time samples were analysed by reversed-phase HPLC as described by R.G. Lauback et at., J. of Liq. Chrom. 7, (1984) 1240.
- Detailed description:: column: µ Bondapak C-18, 3.9 x 150 mm
eluent: 3.5 mM SDS, 32.5% v/v acetonitrile, 0.25 M AcOH, pH 3.0
Detection: 254 nm
Retention times:
6-APA : 3.4 min
Ampi : 9.1 min
D,L-PGMe : 11 min.
A fixed amount of enzyme (1 U/ml) was added.

The time needed to reach the maximum conversion efficiency and the maximum conversion efficiency itself are shown in Table II for the different incubation temperatures.

**TABLE II**

| TEMP (°C) | AMPI FORMED (%) | TIME (hours) |
|---|---|---|
| 5 | 88 | 6.0 |
| 10 | 85 | 4.0 |
| 15 | 85 | 2.0 |
| 20 | 80 | 1.5 |
| 25 | 78 | 0.75 |
| 30 | 73 | 0.5 |
| 35 | 72 | 0.5 |

Essentially the same result was obtained using the D-PG ethyl ester.

## Claims

1. Process for the enzymatic preparation of ampicillin from 6-APA and a D,L-phenylglycine or D-phenylglycine derivative, characterized in that the reaction is carried out at a temperature of 0-15°C.

2. Process according to claim 1, characterized in that the reaction is carried out at a temperature of 5-15°C.

## Patentansprüche

1. Verfahren zur enzymatischen Herstellung von Ampicillin aus 6-APA und einem D,L-Phenylglycin- oder D-Phenylglycinderivat, dadurch gekennzeichnet, daß die Reaktion bei einer Temperatur von 0 bis 15° C durchgeführt wird.

2. Verfahren nach Anspruch 1, dadurch gekennzeichnet, daß die Reaktion bei einer Temperatur von 5 bis 15° C durchgeführt wird.

## Revendications

1. Procédé de préparation enzymatique d'ampicilline à partir de 6-APA et d'un dérivé de la D,L-phénylglycine ou de la D-phénylglycine, caractérisé en ce que la réaction est réalisée à une température de 0 à 15°C.

2. Procédé suivant la revendication 1, caractérisé en ce que la réaction est réalisée à une température de 5 à 15°C.
